# EUROPÄISCHE PATENTSCHRIFT

(11) **EP 1 373 228 B1**
(45) Veröffentlichungstag und Bekanntmachung des Hinweises auf die Patenterteilung: **16.04.2008**
(21) Anmeldenummer: 02719937.1
(22) Anmeldetag: 27.02.2002
(51) Int. Cl.: C07D 251/16, C07D 251/22, C07D 251/46, C07D 239/42, C07D 239/46, A01N 47/36

(54) **SUBSTITUIERTE FLUORALKOXYPHENYLSULFONYLHARNSTOFFE**
SUBSTITUTED FLUOROALCOXYPHENYLSULFONYLUREA
FLUOROALCOXYPHENYLSULFONYLUREES SUBSTITUEES

(30) Priorität: 12.03.2001 DE 10111649
(43) Veröffentlichungstag der Anmeldung: 02.01.2004
(73) Patentinhaber: Bayer CropScience AG, 40789 Monheim (DE)
(72) Erfinder: MÜLLER, Klaus-Helmut, 40593 Düsseldorf (DE); GESING, Ernst-Rudolf, 40699 Erkrath (DE); KLUTH, Joachim, 40764 Langenfeld (DE); DREWES, Mark, Wilhelm, 40764 Langenfeld (DE); DAHMEN, Peter, 41470 Neuss (DE); FEUCHT, Dieter, 40789 Monheim (DE); PONTZEN, Rolf, 42799 Leichlingen (DE); WACHENDORFF-NEUMANN, Ulrike, 56566 Neuwied (DE)
(74) Vertreter: Schwenk, Norbert
(86) Internationale Anmeldenummer: PCT/EP2002/002064
(87) Internationale Veröffentlichungsnummer: WO 2002/072560

(56) Entgegenhaltungen:
- EP-A- 0 044 807
- EP-A- 0 044 808
- EP-A- 0 113 956
- WO-A-93/17001
- WO-A-97/03056
- WO-A-97/32861
- US-A- 4 452 628

## Beschreibung

Die Erfindung betrifft neue substituierte Fluoralkoxyphenylsulfonylharnstoffe, Verfahren zu ihrer Herstellung und ihre Verwendung als Pflanzenbehandlungsmittel, insbesondere als Herbizide und als Fungizide.

Es ist bereits bekannt, dass bestimmte substituierte Sulfonylharnstoffe herbizide Eigenschaften aufweisen (vgl. EP-A-1514, EP-A-23422, EP-A-44807, EP-A-44808, WO-A-97/32861). Die herbizide Wirksamkeit und die Verträglichkeit gegenüber Kulturpflanzen dieser Verbindungen sind jedoch nicht in allen Belangen zufriedenstellend. Eine fungizide Wirksamkeit dieser Verbindungen ist bisher nicht bekannt geworden.
Es wurden nun die neuen substituierten Fluoralkoxyphenylsulfonylharnstoffe der allgemeinen Formel (I) 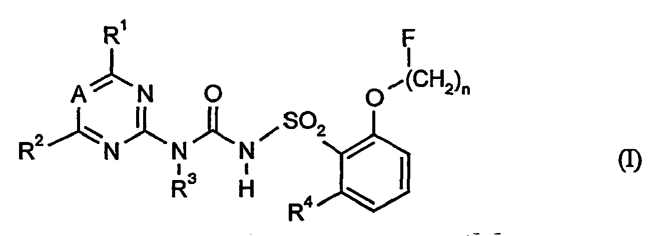 in welcher
- n: für die Zahlen 2, 3 oder 4 steht,
- A: für Stickstoff oder die Gruppierung C-X steht, wobei
X für Wasserstoff, Halogen oder jeweils gegebenenfalls durch Halogen substituiertes Alkyl oder Alkoxy mit jeweils 1 bis 4 Kohlenstoffatomen steht, oder zusammen mit R¹ oder R² für eine der Gruppierungen -CH₂CH₂O-, -CH₂CH₂S-, -CH₂CH₂CH₂- steht,
- R¹: für Wasserstoff, für Halogen oder für jeweils gegebenenfalls durch Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen steht,
- R²: für Wasserstoff, für Cyano, für Halogen, für jeweils gegebenenfalls durch Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für Cycloalkyl mit 3 bis 6 Kohlenstoffatomen steht,
- R³: für Wasserstoff oder für gegebenenfalls durch Cyano, Halogen, C₁-C₄-Alkoxy oder C₁-C₄-Alkoxy-carbonyl substituiertes Alkyl mit 1 bis 4 Kohlenstoffatomen steht, und
- R⁴: für Halogen, für jeweils gegebenenfalls durch Cyano, Halogen oder C₁-C₄-Alkoxy substituiertes Alkyl oder Alkoxy mit jeweils 1 bis 4 Kohlenstoffatomen, für jeweils gegebenenfalls durch Cyano oder Halogen substituiertes Alkenyl, Alkinyl, Alkenyloxy oder Alkinyloxy mit jeweils 2 bis 4 Kohlenstoffatomen, oder für gegebenenfalls durch Cyano, Halogen oder C₁-C₄-Alkyl substituiertes Cycloalkyl mit 3 bis 6 Kohlenstoffatomen steht,
sowie Salze von Verbindungen der Formel (I) gefunden.

Bevorzugte Substituenten bzw. Bereiche der in den oben und nachstehend aufgeführten Formeln vorhandenen Reste werden im folgenden beschrieben:
- n: steht bevorzugt für die Zahlen 2, 3 oder 4.
- A: steht bevorzugt für Stickstoff oder die Gruppierung C-X, wobei
X für Wasserstoff, Fluor, Chlor, Brom oder jeweils gegebenenfalls durch Fluor und/oder Chlor substituiertes Methyl, Ethyl, Methoxy, oder Ethoxy steht, oder zusammen mit R¹ oder R² für eine der Gruppierungen -CH₂CH₂O-, -CH₂CH₂S-, -CH₂CH₂CH₂- steht,
- R¹: steht bevorzugt für Wasserstoff, Fluor, Chlor, Brom, oder für jeweils gegebenenfalls durch Fluor, Chlor, Methoxy, Ethoxy oder n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R²: steht bevorzugt für Wasserstoff, Cyano, Fluor, Chlor, Brom, für jeweils gegebenenfalls durch Fluor, Chlor, Methoxy, Ethoxy oder n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino, oder für Cyclopropyl, Cyclobutyl, Cyclopentyl oder Cyclohexyl.
- R³: steht bevorzugt für Wasserstoff oder für jeweils gegebenenfalls durch Cyano, Fluor, Chlor, Methoxy, Ethoxy, n- oder i-Propoxy, Methoxycarbonyl, Ethoxycarbonyl, n- oder i-Propoxy-carbonyl substituiertes Methyl, Ethyl, n- oder i-Propyl, n-, i-, s- oder t-Butyl.
- R⁴: steht bevorzugt für Fluor, Chlor, Brom, oder für jeweils gegebenenfalls durch Fluor, Chlor, Brom, Methoxy, Ethoxy, n- oder i-Propoxy, n-, i-, s- oder t-Butoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, für jeweils gegebenenfalls durch Fluor, Chlor und/oder Brom substituiertes Ethenyl, Propenyl, Butenyl, Ethinyl, Propinyl, Butinyl, Propenyloxy, Butenyloxy, Propinyloxy oder Butinyloxy, oder für jeweils gegebenenfalls durch Fluor, Chlor oder Methyl substituiertes Cyclopropyl, Cyclobutyl oder Cyclopentyl.
- n: steht besonders bevorzugt für die Zahlen 2, 3 oder 4.
- A: steht besonders bevorzugt für Stickstoff oder die Gruppierung C-X, wobei
X für Wasserstoff, Fluor, Chlor, Methyl oder Methoxy steht, oder zusammen mit R¹ oder R² für eine der Gruppierungen -CH₂CH₂O-, -CH₂CH₂S-, -CH₂CH₂CH₂- steht
- R¹: steht besonders bevorzugt für Wasserstoff, Fluor, Chlor, Brom, oder für jeweils gegebenenfalls durch Fluor, Chlor, Methoxy, Ethoxy oder n- oder i-Propoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino.
- R²: steht besonders bevorzugt für Wasserstoff Cyano, Fluor, Chlor, Brom, für jeweils gegebenenfalls durch Fluor, Chlor, Methoxy, Ethoxy oder n- oder i-Propoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino, oder für Cyclopropyl.
- R³: steht besonders bevorzugt für Wasserstoff oder für jeweils gegebenenfalls durch Cyano, Fluor, Chlor, Methoxy, Ethoxy, n- oder i-Propoxy, Methoxycarbonyl, Ethoxycarbonyl, n- oder i-Propoxy-carbonyl substituiertes Methyl, Ethyl, n- oder i-Propyl.
- R⁴: steht besonders bevorzugt für Fluor, Chlor, Brom, oder für jeweils gegebenenfalls durch Fluor, Chlor, Methoxy, Ethoxy, n- oder i-Propoxy substituiertes Methyl, Ethyl, n- oder i-Propyl, Methoxy, Ethoxy, n- oder i-Propoxy, für jeweils gegebenenfalls durch Fluor, Chlor und/oder Brom substituiertes Propenyl, Butenyl, Propinyl, Butinyl, Propenyloxy, Butenyloxy, Propinyloxy oder Butinyloxy, oder für gegebenenfalls durch Fluor, Chlor oder Methyl substituiertes Cyclopropyl.
- n: steht ganz besonders bevorzugt für die Zahlen 2 oder 3.
- A: steht ganz besonders bevorzugt für Stickstoff oder eine CH-Gruppierung.
- R¹: steht ganz besonders bevorzugt für Cyano, Chlor, Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamino, Ethylamino, Dimethylamino oder Cyclopropyl.
- R²: steht ganz besonders bevorzugt für Chlor, Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamino, Ethylamino oder Dimethylamino.
- R³: steht ganz besonders bevorzugt für Wasserstoff oder Methyl.
- R⁴: steht ganz besonders bevorzugt für Fluor, Chlor, Brom, Methyl, Trifluormethyl, Ethyl, n- oder i-Propyl, Methoxy, Ethoxy, n- oder i-Propoxy, oder für Cyclopropyl.

Eine ganz besonders bevorzugte Gruppe sind diejenigen Verbindungen der Formel (I), in welcher
- n: für die Zahlen 2 oder 3 steht,
- A: für Stickstoff steht,
- R¹: für Chlor, Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamino, Ethylamino oder Dimethylamino steht,
- R²: für Chlor, Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamino, Ethylamino oder Dimethylamino steht,
- R³: für Wasserstoff oder Methyl steht, und
- R⁴: für Fluor, Chlor, Brom, Methyl, Trifluormethyl, Ethyl, n- oder i-Propyl, Methoxy, Ethoxy, n- oder i-Propoxy steht.

Eine weitere ganz besonders bevorzugte Gruppe sind diejenigen Verbindungen der Formel (I), in welcher
- n: für die Zahlen 2 oder 3 steht,
- A: für eine CH-Gruppierung steht,
- R¹: für Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamino, Ethylamino oder Dimethylamino steht,
- R²: für Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamin, Ethylamino oder Dimethylamino steht,
- R³: für Wasserstoff oder Methyl steht, und
- R⁴: für Fluor, Chlor, Brom, Methyl, Trifluormethyl, Ethyl, n- oder i-Propyl, Methoxy, Ethoxy, n- oder i-Propoxy steht.

Als eine weitere erfindungsgemäß bevorzugte Gruppe seien die Verbindungen erwähnt, bei denen n für 3 oder 4 steht.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher n, A, R¹, R², R³ und R⁴ die oben vorzugsweise angegebenen Bedeutungen haben.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Erfindungsgemäß bevorzugt sind diejenigen Verbindungen der Formel (I), bei welchen eine Kombination der vorstehend als bevorzugt ("vorzugsweise") aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Erfindungsgemäß am meisten bevorzugt sind diejenigen Verbindungen der Formel (I), bei welchen eine Kombination der vorstehend als am meisten bevorzugt aufgeführten Bedeutungen vorliegt.

Gesättigte oder ungesättigte Kohlenwasserstoffreste, wie Alkyl oder Alkenyl, sind - auch in Verbindung mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.

Die neuen substituierten Fluoralkoxyphenylsulfonylharnstoffe der allgemeinen Formel (I) weisen interessante biologische Eigenschaften auf. Sie zeichnen sich insbesondere durch starke herbizide und fungizide Wirksamkeit aus.

Man erhält die neuen substituierten Fluoralkoxyphenylsulfonylharnstoffe der allgemeinen Formel (I), wenn man substituierte Aminoazine der allgemeinen Formel (II) 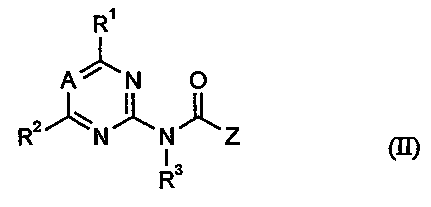 in welcher
A, R¹ und R² die oben angegebene Bedeutung haben,
Z für Halogen, Alkoxy oder Aryloxy steht und
R³ die oben angegebene Bedeutung hat oder für die Gruppierung -C(O)-Z steht,
mit Fluoralkoxybenzolsulfonamiden der allgemeinen Formel (III) 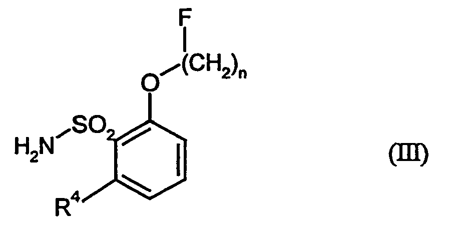 in welcher
n und R⁴ die oben angegebene Bedeutung haben,
gegebenenfalls in Gegenwart eines oder mehrerer Reaktionshilfsmittel und gegebenenfalls in Gegenwart eines oder mehrerer Verdünnungsmittel umsetzt,
und gegebenenfalls die so erhaltenen Verbindungen der allgemeinen Formel (I) nach üblichen Methoden in ihre Salze überfährt.

Die neuen substituierten Fluoralkoxyphenylsulfonylharnstoffe der allgemeinen Formel (I) können prinzipiell auch wie im Folgenden schematisch dargestellt erhalten werden:
(b) durch Umsetzung von Aminoazinen der allgemeinen Formel (IV) mit Fluoralkoxyphenylsulfonylisocyanaten der allgemeinen Formel (V); n, R¹, R², R³ und R⁴ wie oben (vgl. WO-A-97/32861): 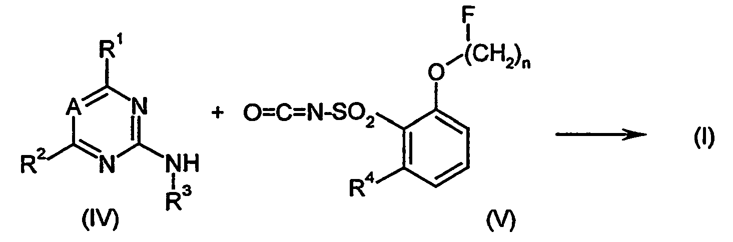
(c) durch Umsetzung von Aminoazinen der allgemeinen Formel (IV) mit substituierten Fluoralkoxybenzolsulfonamiden der allgemeinen Formel (VI); n, R¹, R², R³ und R⁴ wie oben, Z: Halogen, C₁-C₄-Alkoxy oder Phenoxy (vgl. WO-A-97/32861): 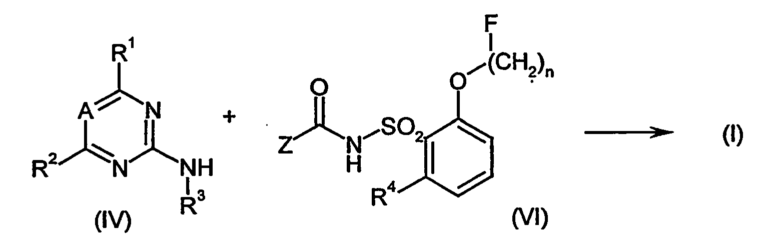
Verwendet man beispielsweise 2-Methoxycarbonylamino-4-methoxy-6-trifluormethyl-1,3,5-triazin und 2-(2-Fluor-ethoxy)-6-methyl-benzolsulfonamid als Ausgangsstoffe, so kann der Reaktionsablauf beim erfindungsgemäßen Verfahren durch das folgende Formelschema skizziert werden:

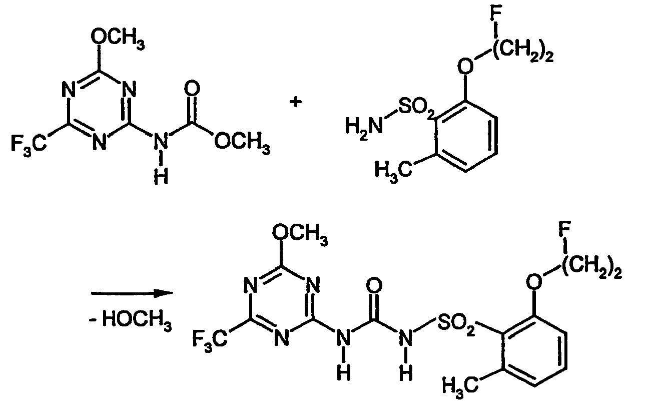

Die beim erfindungsgemäßen Verfahren zur Herstellung von Verbindungen der allgemeinen Formel (I) als Ausgangsstoffe zu verwendenden Aminoazine sind durch die Formel (II) allgemein definiert. In der allgemeinen Formel (II) haben A, R¹ und R² vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für A, R¹ und R² angegeben worden sind; Z steht vorzugsweise für Fluor, Chlor, Brom, C₁-C₄₋Alkoxy oder Phenoxy, insbesondere für Chlor, Methoxy, Ethoxy oder Phenoxy.

Die Ausgangsstoffe der allgemeinen Formel (II) sind bekannt und/oder können nach an sich bekannten Verfahren hergestellt werden (vgl. DE-A-19 501 174, US-A-4 690 707).

Die beim erfindungsgemäßen Verfahren zur Herstellung von Verbindungen der allgemeinen Formel (I) weiter als Ausgangsstoffe zu verwendenden Fluoralkoxybenzolsulfonamide sind durch die Formel (III) allgemein definiert. In der allgemeinen Formel (III) haben n und R⁴ vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für n und R⁴ angegeben worden sind.

Die Ausgangsstoffe der allgemeinen Formel (III) sind teilweise durch Offenbarung in Form einer generischen Formel bekannt (vgl WO 97/03056). Die Ausgangstoffe der Formel (III) worin n=3, und R⁴ die in Anspruch 1 angegebene Bedeutung hat, stellen jedoch eine neue Auswahl aus den vorbekannten Verbindungen dar, und sind deshalb auch Gegenstand der vorliegenden Anmeldung.

Man erhält die neuen Fluoralkoxybenzolsulfonamide der allgemeinen Formel (III), wenn man Hydroxybenzolsulfonamide der allgemeinen Formel (VII) 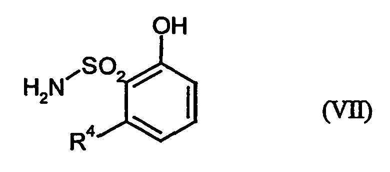 in welcher
- R⁴: die oben angegebene Bedeutung hat,
mit mit ω-Fluor-α-halogen-alkanen der allgemeinen Formel (VIII) 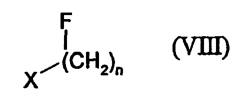 in welcher
- n: die oben angegebene Bedeutung hat und
- X: für Halogen, bevorzugt für Chlor, Brom oder Iod, insbesondere für Brom, oder für Methylsulfonyloxy, Phenylsulfonyloxy oder Tolylsulfonyloxy steht,

gegebenenfalls in Gegenwart eines Verdünnungsmittels, wie z.B. Aceton, Butanon, Acetonitril, Propionitril, N,N-Dimethyl-formamid oder N,N-Dimethyl-acetamid, und gegebenenfalls in Gegenwart eines Säureakzeptors, wie z.B. Kaliumcarbonat, bei Temperaturen zwischen 0°C und 150°C umsetzt (vgl. die Herstellungsbeispiele).

Die als Vorprodukte benötigten Hydroxybenzolsulfonamide der allgemeinen Formel (VII) sind bekannt und/oder können nach an sich bekannten Verfahren hergestellt werden (vgl. EP-A-44807, WO-A-97/03056).

Die weiter als Vorprodukte benötigten ω-Fluor-α-halogen-alkanen der allgemeinen Formel (VIII) sind bekannte organische Synthesechemikalien.

Das erfindungsgemäße Verfahren zur Herstellung der neuen substituierten Fluoralkoxyphenylsulfonylharnstoffe der allgemeinen Formel (I) wird vorzugsweise in Gegenwart eines oder mehrerer Reaktionshilfsmittel durchgeführt. Als Reaktionshilfsmittel für das erfindungsgemäße Verfahren kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calciumamid, Natrium-, Kalium- oder Calcium-carbonat, Natrium-, Kalium- oder Calciumhydrogencarbonat, Lithium-, Natrium-, Kalium- oder Calcium-hydrid, Lithium-, Natrium-, Kalium- oder Calcium-hydroxid, Natrium- oder Kalium- -methanolat, - ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2.2.2]-octan (DABCO), 1,5-Diazabicyclo[4.3.0]-non-5-en (DBN), oder 1,8 Diazabicyclo[5.4.0]-undec-7-en (DBU).

Als weitere Reaktionshilfsmittel für das erfindungsgemäße Verfahren kommen auch Phasentransfer-Katalysatoren in Betracht. Als Beispiele für solche Katalysatoren seien genannt:
Tetrabutylammonium-bromid, Tetrabutylammonium-chlorid, Tetraoctylammoniumchlorid, Tetrabutylammonium hydrogensulfat, Methyl-trioctylammonium-chlorid, Hexadecyl-trimethylammonium-chlorid, Hexadecyl-trimethylammonium-bromid, Benzyl-trimethylammonium-chlorid, Benzyl-triethylammonium-chlorid, Benzyl-trimethylammonium-hydroxid, Benzyl-triethylammonium-hydroxid, Benzyl-tributylammonium-chlorid, Benzyl-tributylammonium-bromid, Tetrabutylphosphoniumbromid, Tetrabutylphosphonium-chlorid, Tributyl-hexadecylphosphonium-bromid, Butyl-triphenylphosphonium-chlorid, Ethyl-trioctylphosphonium-bromid, Tetraphenylphosphonium-bromid.

Das erfindungsgemäße Verfahren zur Herstellung der Verbindungen der allgemeinen Formel (I) wird vorzugsweise unter Verwendung eines oder mehrerer Verdünnungsmittel durchgeführt. Als Verdünnungsmittel zur Durchführung des erfindungsgemäßen Verfahrens kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Düsopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base, wie z.B. Natriumhydroxid. Die Salze können dann - gegebenenfalls nach längerem Rühren - durch Einengen oder Absaugen isoliert werden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie weisen in gewissem Umfang auch resistenz-induzierende Wirkung auf. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Mont morillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenem") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Amitrole, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromacil, Bromobutide, Bromofenoxim, Bromoxynil, Butachlor, Butafenacil (-allyl), Butenachlor, Butralin, Butroxydim, Butylate, Cafenstrole, Carbetamide, Carfentrazone (-ethyl), Chlomethoxyfen, Chloramben, Chloridazon, Chlorimuron (-ethyl), Chlornitrofen, Chlorsulfuron, Chlorthiamid, Chlortoluron, Cinidon (-ethyl), Cinmethylin, Cinosulfuron, Clefoxydim, Clethodim, Clodinafop (-propargyl), Clomazone, Clomeprop, Clopyralid, Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlobenil, Dichlorprop (-P), Diclofop (-methyl), Diclosulam, Diethatyl (-ethyl), Difenopenten (-ethyl), Difenzoquat, Diflufenican, Diflufenzopyr, Dikegulac (-sodium), Dimefuron, Dimepiperate, Dimethachlor, Dimethametryn, Dimethenamid (-P), Dimexyflam, Dinitramine, Diphenamid, Diquat (-dibromide), Dithiopyr, Diuron, Dymron, Epropodan, EPTC, Esprocarb, Ethalfluralin, Ethametsulfuron (-methyl), Ethiozin, Ethofumesate, Ethoxyfen, Ethoxysulfuron, Etobenzanid, Fenoxaprop (-P-ethyl), Fentrazamide, Flamprop (-M-isopropyl, -M-methyl), Flazasulfuron, Florasulam, Fluazifop (-P-butyl), Fluazolate, Flucarbazone (-sodium), Fluchloralin, Flufenacet, Flumetsulam, Flumiclorac (-pentyl), Flumioxazin, Flumipropyn, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fomesafen, Foramsulfuron, Glufosinate (-ammonium), Glyphosate (-ammonium, -isopropylammonium), Halosafen, Halosulfuron (-methyl), Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxadifen (-ethyl), Isoxaflutole, Isoxapyrifop, Ketospiradox, Lactofen, Lenacil, Linuron, MCPA, Mecoprop (-P), Mefenacet, Mesotrione, Metamitron, Metazachlor, Methabenzthiazuron, Methyldymron, Metobenzuron, Metobromuron, (S-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Pethoxamid, Phenmedipham, Picolinafen, Piperophos, Pretilachlor, Primisulfuron (-methyl), Profluazol, Profoxydim, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridafol, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifloxysulfuron, Trifluralin, Triflusulfuron (-methyl), Tritosulfuron.

Für die Mischungen kommen weiterhin bekannte Safener in Frage, beispielsweise AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematizide, Schutzstoffen gegen Vogelfraß, Pflanzennährstoffen und Bodenstrulcturverbesserungsmitteln ist möglich.

Die Wirkstoffe können als solche, in Form ihrer Formulierungen oder den daraus durch weiteres Verdünnen bereiteten Anwendungsfomaen, wie gebrauchsfertige Lösungen, Suspensionen, Emulsionen, Pulver, Pasten und Granulate angewandt werden.

Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

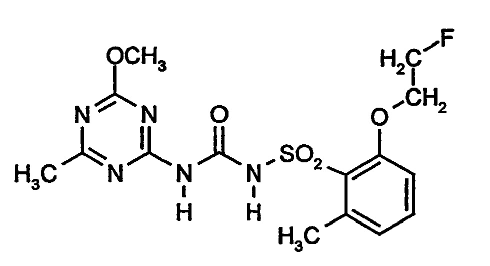

Eine Lösung von 23,4 g (154 mMol) 1,8 Diazabicyclo[5.4.0]-undec-7-en (DBU) in 50 ml Acetonitril wird bei Raumtemperatur (ca. 20°C) tropfenweise unter Rühren zu einer Mischung aus 32,7 g (140 mMol) 2-(2-Fluor-ethoxy)-6-methyl-benzolsulfonamid, 53,2 g (140 mMol) 2-(N,N-Bis-phenoxycarbonyl-amino)-4-methoxy-6-methyl-1,3,5-triazin und 300 ml Acetonitril gegeben. Die Reaktionsmischung wird eine Stunde bei Raumtemperatur gerührt und dann unter vermindertem Druck eingeengt. Der Rückstand wird in 300 ml Methylenchlorid aufgenommen, mit 1N-Salzsäure, dann mit Wasser und schließlich mit gesättigter wässriger Natriumchlorid-Lösung geschüttelt. Die organische Phase wird dann mit Natriumsulfat getrocknet und filtriert. Das Filtrat wird eingeengt und der Rückstand mit 100 ml Diethylether digeriert. Das kristallin angefallene Produkt wird durch Absaugen isoliert.

Man erhält 36,9 g (60 % der Theorie) N-[2-(2-Fluor-ethoxy)-6-methyl-phenylsulfonyl]-N'-(4-methoxy-6-methyl-1,3,5-triazin-2-yl)-harnstoff vom Schmelzpunkt 161°C.

### Beispiel 2

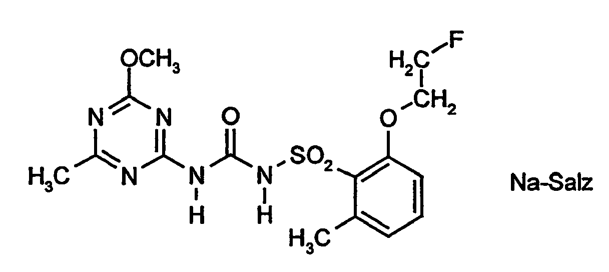

3,3 g (82 mMol) Natriumhydroxid (Micropills) werden bei Raumtemperatur unter Rühren zu einer Mischung aus 36,0 g (82 mMol) N-[2-(2-Fluor-ethoxy)-6-methylphenylsulfonyl]-N'-(4-methoxy-6-methyl-1,3,5-triazin-2-yl)-harnstoff und 250 ml Methylenchlorid gegeben. Die Mischung wird etwa 20 Stunden bei Raumtemperatur gerührt; dann wird das kristallin angefallene Produkt durch Absaugen isoliert.

Man erhält 36,9 g (100 % der Theorie) N-[2-(2-Fluor-ethoxy)-6-methyl-phenylsulfonyl]-N'-(4-methoxy-6-methyl-1,3,5-triazin-2-yl)-harnstoff-Natriumsalz vom Schmelzpunkt 185°C.

Analog zu den Beispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

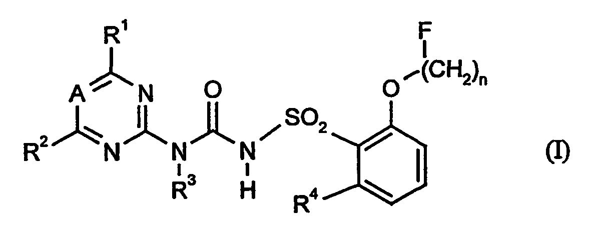

**Tabelle 1: Beispiele für die Verbindungen der Formel (I)**

| Bsp.- | | | | | | | Physikal. |
|---|---|---|---|---|---|---|---|
| Nr. | n | A | R¹ | R² | R³ | R⁴ | Daten |
| 3 | 2 | N | CH₃ | OCH₃ | H | CF₃ | Fp.: 124°C |
| 4 | 2 | CH | OCH₃ | OCH₃ | H | CF₃ | Fp.: 183°C |
| 5 | 2 | N | CH₃ | OCH₃ | H | CF₃ | Fp.: 187°C |
| | | | | | | | (Na-Salz) |
| 6 | 3 | CH | CH₃ | CH₃ | H | C₂H₅ | Fp.:213°C |
| 7 | 3 | N | CH₃ | OCH₃ | H | C₃H₇-n | Fp.: 109°C |
| 8 | 3 | N | OCH₃ | OCH₃ | H | C₃H₇-n | Fp.: 139°C |
| 9 | 3 | N | OCH₂CF₃ | N(CH₃)₂ | H | CH₃ | Fp.: 180°C |
| 10 | 3 | N | OCH₂CF₃ | N(CH₃)₂ | H | C₂H₅ | Fp.: 113°C |
| 11 | 3 | N | OCH₂CF₃ | N(CH₃)₂ | H | C₃H₇-n | Fp.: 172°C |
| 12 | 3 | CH | OCH₃ | OCH₃ | H | CH₃ | Fp.: 142°C |
| 13 | 3 | N | CH₃ | OCH₃ | H | CH₃ | Fp.: 170°C |
| 14 | 3 | CH | Cl | OCH₃ | H | CH₃ | Fp.: 163°C |
| 15 | 3 | N | OCH₃ | OCH₃ | H | CH₃ | Fp.: 155°C |
| 16 | 3 | CH | OCH₃ | OCH₃ | H | C₂H₅ | Fp.: 154°C |
| 17 | 3 | N | CH₃ | OCH₃ | H | C₂H₅ | Fp.: 121 °C |
| 18 | 3 | CH | Cl | OCH₃ | H | C₂H₅ | Fp.: 139°C |
| 19 | 3 | N | OCH₃ | OCH₃ | H | C₂H₅ | Fp.: 130°C |
| 20 | 2 | CH | Cl | OCH₃ | H | CF₃ | Fp.: 123°C |
| 21 | 2 | N | OCH₃ | OCH₃ | H | CF₃ | Fp.: 169°C |
| 22 | 2 | N | OCH₂CF₃ | N(CH₃)₂ | H | CF₃ | Fp.: 162°C |
| 23 | 2 | N | OCH₃ | OCH₃ | CH₃ | CF₃ | Fp.: 176°C |
| 24 | 2 | CH | OCH₃ | OCH₃ | H | CH₃ | Fp.: 169°C |
| 25 | 3 | CH | OCH₃ | OCH₃ | H | C₃H₇-n | Fp.: 114°C |
| 26 | 2 | CH | OCH₃ | OCH₃ | H | C₃H₇-n | Fp.: 166°C |
| 27 | 2 | N | CH₃ | OCH₃ | CH₃ | CF₃ | Fp.: 177°C |
| 28 | 3 | N | CH₃ | OCH₃ | CH₃ | C₂H₅ | Fp.: 124°C |
| 29 | 3 | N | CH₃ | OCH₃ | CH₃ | CH₃ | Fp.: 146°C |
| 30 | 3 | N | OCH₃ | OCH₃ | CH₃ | C₂H₅ | Fp.:168°C |
| 31 | 3 | N | OCH₃ | OCH₃ | CH₃ | C₃H₇-n | Fp.:150°C |
| 32 | 3 | N | OCH₃ | OCH₃ | CH₃ | CH₃ | Fp.:165°C |
| 33 | 2 | CH | CH₃ | CH₃ | H | CH₃ | |
| 34 | 2 | N | CH₃ | CH₃ | H | CH₃ | |
| 35 | 2 | CH | CH₃ | OCH₃ | H | CH₃ | |
| 36 | 2 | N | OCH₃ | OCH₃ | H | CH₃ | |
| 37 | 2 | N | OCH₂CF₃ | N(CH₃)2 | H | CH₃ | |
| 38 | 2 | CH | Cl | OCH₃ | H | CH₃ | |
| 39 | 2 | CH | CH₃ | CH₃ | H | C₂H₅ | |
| 40 | 2 | N | CH₃ | CH₃ | H | C₂H₅ | |
| 41 | 2 | CH | CH₃ | OCH₃ | H | C₂H₅ | |
| 42 | 2 | N | CH₃ | OCH₃ | H | C₂H₅ | |
| 43 | 2 | CH | OCH₃ | OCH₃ | H | C₂H₅ | |
| 44 | 2 | N | OCH₃ | OCH₃ | H | C₂H₅ | |
| 45 | 2 | N | OCH₂CF₃ | N(CH₃)₂ | H | C₂H₅ | |
| 46 | 2 | CH | Cl | OCH₃ | H | C₂H₅ | |
| 47 | 2 | CH | CH₃ | CH₃ | H | C₃H₇-n | |
| 48 | 2 | N | CH₃ | CH₃ | H | C₃H₇-n | |
| 49 | 2 | CH | CH₃ | OCH₃ | H | C₃H₇-n | |
| 50 | 2 | N | CH₃ | OCH₃ | H | C₃H₇-n | |
| 51 | 2 | N | OCH₃ | OCH₃ | H | C₃H₇-n | |
| 52 | 2 | N | OCH₂CF₃ | N(CH₃)₂ | H | C₃H₇-n | |
| 53 | 2 | CH | Cl | OCH₃ | H | C₃H₇-n | |
| 54 | 2 | CH | CH₃ | CH₃ | H | C₃H₇-i | |
| 55 | 2 | N | CH₃ | CH₃ | H | C₃H₇-i | |
| 56 | 2 | CH | CH₃ | OCH₃ | H | C₃H₇-i | |
| 57 | 2 | N | CH₃ | OCH₃ | H | C₃H₇-i | |
| 58 | 2 | CH | OCH₃ | OCH₃ | H | C₃H₇-i | |
| 59 | 2 | N | OCH₃ | OCH₃ | H | C₃H₇-i | |
| 60 | 2 | N | OCH₂CF₃ | N(CH₃)₂ | H | C₃H₇-i | |
| 61 | 2 | CH | Cl | OCH₃ | H | C₃H₇-i | |
| 62 | 2 | CH | CH₃ | CH₃ | H | CF₃ | Fp.:168°C |
| 63 | 2 | N | CH₃ | CH₃ | H | CF₃ | |
| 64 | 2 | CH | CH₃ | OCH₃ | H | CF₃ | Fp.:176°C |
| 65 | 2 | CH | CH₃ | CH₃ | H | Br | |
| 66 | 2 | N | CH₃ | CH₃ | H | Br | |
| 67 | 2 | CH | CH₃ | OCH₃ | H | Br | |
| 68 | 2 | N | CH₃ | OCH₃ | H | Br | |
| 69 | 2 | CH | OCH₃ | OCH₃ | H | Br | |
| 70 | 2 | N | OCH₃ | OCH₃ | H | Br | |
| 71 | 2 | N | OCH₂CF₃ | N(CH₃)₂ | H | Br | |
| 72 | 2 | CH | Cl | OCH₃ | H | Br | |
| 73 | 2 | CH | CH₃ | CH₃ | H | OCH₃ | |
| 74 | 2 | N | CH₃ | CH₃ | H | OCH₃ | |
| 75 | 2 | CH | CH₃ | OCH₃ | H | OCH₃ | |
| 76 | 2 | N | CH₃ | OCH₃ | H | OCH₃ | |
| 77 | 2 | CH | OCH₃ | OCH₃ | H | OCH₃ | |
| 78 | 2 | N | OCH₃ | OCH₃ | H | OCH₃ | |
| 79 | 2 | N | OCH₂CF₃ | N(CH₃)₂ | H | OCH₃ | |
| 80 | 2 | CH | Cl | OCH₃ | H | OCH₃ | |
| 81 | 2 | CH | CH₃ | CH₃ | H | OC₂H₅ | |
| 82 | 2 | N | CH₃ | CH₃ | H | QC₂H₅ | |
| 83 | 2 | CH | CH₃ | OCH₃ | H | OC₂H₅ | |
| 84 | 2 | N | CH₃ | OCH₃ | H | OC₂H₅ | |
| 85 | 2 | CH | OCH₃ | OCH₃ | H | OC₂H₅ | |
| 86 | 2 | N | OCH₃ | OCH₃ | H | OC₂H₅ | |
| 87 | 2 | N | OCH₂CF₃ | N(CH₃)₂ | H | OC₂H₅ | |
| 88 | 2 | CH | Cl | OCH₃ | H | OC₂H₅ | |
| 89 | 3 | CH | CH₃ | CH₃ | H | CH₃ | |
| 90 | 3 | N | CH₃ | CH₃ | H | CH₃ | |
| 91 | 3 | CH | CH₃ | OCH₃ | H | CH₃ | |
| 92 | 3 | N | CH₃ | CH₃ | H | C₂H₅ | |
| 93 | 3 | CH | CH₃ | OCH₃ | H | C₂H₅ | |
| 94 | 3 | CH | CH₃ | CH₃ | H | C₃H₇-n | |
| 95 | 3 | N | CH₃ | CH₃ | H | C₃H₇-n | |
| 96 | 3 | CH | CH₃ | OCH₃ | H | C₃H₇-n | |
| 97 | 3 | CH | Cl | OCH₃ | H | C₃H₇-n | |
| 98 | 3 | CH | CH₃ | CH₃ | H | C₃H₇-i | |
| 99 | 3 | N | CH₃ | CH₃ | H | C₃H₇-i | |
| 100 | 3 | CH | CH₃ | OCH₃ | H | C₃H₇-i | |
| 101 | 3 | N | CH₃ | OCH₃ | H | C₃H₇-i | |
| 102 | 3 | N | CH₃ | OCH₃ | CH₃ | C₃H₇-i | |
| 103 | 3 | CH | OCH₃ | OCH₃ | H | C₃H₇-i | |
| 104 | 3 | N | OCH₃ | OCH₃ | H | C₃H₇-i | |
| 105 | 3 | N | OCH₂CF₃ | N(CH₃)₂ | H | C₃H₇-i | |
| 106 | 3 | CH | Cl | OCH₃ | H | C₃H₇-i | |
| 107 | 3 | CH | CH₃ | CH₃ | H | CF₃ | |
| 108 | 3 | N | CH₃ | CH₃ | H | CF₃ | |
| 109 | 3 | CH | CH₃ | OCH₃ | H | CF₃ | |
| 110 | 3 | N | CH₃ | OCH₃ | H | CF₃ | Fp.:68°C |
| 111 | 3 | N | CH₃ | OCH₃ | CH₃ | CF₃ | Fp.: 113°C |
| 112 | 3 | CH | OCH₃ | OCH₃ | H | CF₃ | Fp.:189°C |
| 113 | 3 | N | OCH₃ | OCH₃ | H | CF₃ | Fp.:138°C |
| 114 | 3 | N | OCH₂CF₃ | N(CH₃)₂ | H | CF₃ | |
| 115 | 3 | CH | Cl | OCH₃ | H | CF₃ | |
| 116 | 3 | CH | CH₃ | CH₃ | H | Br | |
| 117 | 3 | N | CH₃ | CH₃ | H | Br | |
| 118 | 3 | N | CH₃ | OCH₃ | H | Br | |
| 119 | 3 | CH | CH₃ | OCH₃ | H | Br | |
| 120 | 3 | N | CH₃ | OCH₃ | CH₃ | Br | |
| 121 | 3 | CH | OCH₃ | OCH₃ | H | Br | |
| 122 | 3 | N | OCH₃ | OCH₃ | H | Br | |
| 123 | 3 | N | OCH₂CF₃ | N(CH₃)₂ | H | Br | |
| 124 | 3 | CH | Cl | OCH₃ | H | Br | |
| 125 | 3 | CH | CH₃ | CH₃ | H | OCH₃ | |
| 126 | 3 | N | CH₃ | CH₃ | H | OCH₃ | |
| 127 | 3 | CH | CH₃ | OCH₃ | H | OCH₃ | |
| 128 | 3 | N | CH₃ | OCH₃ | H | OCH₃ | |
| 129 | 3 | N | CH₃ | OCH₃ | CH₃ | OCH₃ = | |
| 130 | 3 | CH | OCH₃ | OCH₃ | H | OCH₃ | |
| 131 | 3 | N | OCH₃ | OCH₃ | H | OCH₃ | |
| 132 | 3 | N | OCH₂CF₃ | N(CH₃)₂ | H | OCH₃ | |
| 133 | 3 | CH | Cl | OCH₃ | H | OC₂H₅ | |
| 134 | 3 | CH | CH₃ | CH₃ | H | OC₂H₅ | |
| 135 | 3 | N | CH₃ | CH₃ | H | OC₂H₅ | |
| 136 | 3 | N | CH₃ | OCH₃ | H | OC2H₅ | |
| 137 | 3 | N | CH₃ | OCH₃ | CH₃ | OC₂H₅ | |
| 138 | 3 | CH | CH₃ | OCH₃ | H | OC₂H₅ | |
| 139 | 3 | CH | OCH₃ | OCH₃ | H | OC₂H₅ | |
| 140 | 3 | N | OCH₃ | OCH₃ | H | OC₂H₅ | |
| 141 | 3 | N | OCH₂CF₃ | N(CH₃)₂ | H | OC₂H₅ | |
| 142 | 3 | CH | Cl | OCH₃ | H | OC₂H₅ | |
| 143 | 4 | N | CH₃ | OCH₃ | H | CH₃ | |
| 144 | 4 | CH | CH₃ | OCH₃ | H | CH₃ | |
| 145 | 4 | N | CH₃ | OCH₃ | CH₃ | CH₃ | |
| 146 | 4 | CH | OCH₃ | OCH₃ | H | CH₃ | |
| 147 | 4 | N | OCH₃ | OCH₃ | H | CH₃ | |
| 148 | 4 | CH | Cl | OCH₃ | H | CH₃ | |
| 149 | 4 | CH | CH₃ | OCH₃ | H | C₂H₅ | |
| 150 | 4 | N | CH₃ | OCH₃ | H | C₂H₅ | |
| 151 | 4 | N | CH₃ | OCH₃ | CH₃ | C₂H₅ | |
| 152 | 4 | CH | CH₃ | OCH₃ | H | C₂H₅ | |
| 153 | 4 | CH | Cl | OCH₃ | H | C₂H₅ | |
| 154 | 4 | CH | CH₃ | OCH₃ | H | C₃H₇-n | |
| 155 | 4 | N | CH₃ | OCH₃ | H | C₃H₇-n | |
| 156 | 4 | N | CH₃ | OCH₃ | CH₃ | C₃H₇-n | |
| 157 | 4 | CH | OCH₃ | OCH₃ | H | C₃H₇-n | |
| 158 | 4 | N | OCH₃ | OCH₃ | H | C₃H₇-n | |
| 159 | 4 | CH | OCH₃ | OCH₃ | H | C₂H₅ | |
| 160 | 4 | N | OCH₃ | OCH₃ | H | C₂H₅ | |
| 161 | 4 | CH | CH₃ | CH₃ | H | CH₃ | |
| 162 | 4 | CH | CH₃ | CH₃ | H | C₂H₅ | |
| 163 | 4 | CH | CH₃ | CH₃ | H | C₃H₇-n | |
| 164 | 4 | CH | CH₃ | CH₃ | H | C₃H₇-i | |
| 165 | 4 | N | CH₃ | OCH₃ | H | C₃H₇-i | |
| 166 | 4 | N | CH₃ | OCH₃ | CH₃ | C₃H₇-i | |
| 167 | 4 | CH | CH₃ | OCH₃ | H | C₃H₇-i | |
| 168 | 4 | CH | OCH₃ | OCH₃ | H | C₃H₇-i | |
| 169 | 4 | N | OCH₃ | OCH₃ | H | C₃H₇-i | |
| 170 | 4 | CH | Cl | OCH₃ | H | C₃H₇-i | |
| 171 | 4 | CH | CH₃ | CH₃ | H | CF₃ | |
| 172 | 4 | N | CH₃ | CH₃ | H | CF₃ | |
| 173 | 4 | CH | CH₃ | OCH₃ | H | CF₃ | |
| 174 | 4 | N | CH₃ | OCH₃ | H | CF₃ | |
| 175 | 4 | N | CH₃ | OCH₃ | CH₃ | CF₃ | |
| 176 | 4 | CH | OCH₃ | OCH₃ | H | CF₃ | |
| 177 | 4 | N | OCH₃ | OCH₃ | H | CF₃ | |
| 178 | 4 | CH | Cl | OCH₃ | H | CF₃ | |
| 179 | 2 | CH | OCHF₂ | OCHF₂ | H | CH₃ | |
| 180 | 2 | CH | OCHF₂ | OCHF₂ | H | C₂H₅ | |
| 181 | 2 | CH | OCHF₂ | OCHF₂ | H | C₃H₇-n | |
| 182 | 2 | CH | OCHF₂ | OCHF₂ | H | C₃H₇-i | |
| 183 | 2 | CH | OCHF₂ | OCHF₂ | H | CF₃ | |
| 184 | 2 | CH | OCHF₂ | OCHF₂ | H | Br | |
| 185 | 2 | CH | OCHF₂ | OCHF₂ | H | OCH₃ | |
| 186 | 2 | CH | OCHF₂ | OCHF₂ | H | OC₂H₅ | |
| 187 | 3 | CH | OCHF₂ | OCHF₂ | H | CH₃ | |
| 188 | 3 | CH | OCHF₂ | OCHF₂ | H | C₂H₅ | |
| 189 | 3 | CH | OCHF₂ | OCHF₂ | H | C₃H₇-n | |
| 190 | 3 | CH | OCHF₂ | OCHF₂ | H | C₃H₇-i | |
| 191 | 3 | CH | OCHF₂ | OCHF₂ | H | CF₃ | |
| 192 | 3 | CH | OCHF₂ | OCHF₂ | H | Br | |
| 193 | 3 | CH | OCHF₂ | OCHF₂ | H | OCH₃ | |
| 194 | 3 | CH | OCHF₂ | OCHF₂ | H | OC₂H₅ | |
| 195 | 4 | CH | OCHF₂ | OCHF₂ | H | CH₃ | |
| 196 | 4 | CH | OCHF₂ | OCHF₂ | H | C₂H₅ | |
| 197 | 4 | CH | OCHF₂ | OCHF₂ | H | C₃H₇-n | |
| 198 | 4 | CH | OCHF₂ | OCHF₂ | H | C₃H₇-i | |
| 199 | 4 | CH | OCHF₂ | OCHF₂ | H | CF₃ | |
| 200 | 4 | CH | OCHF₂ | OCHF₂ | H | Br | |
| 201 | 4 | CH | OCHF₂ | OCHF₂ | H | OCH₃ | |
| 202 | 4 | CH | OCHF₂ | OCHF₂ | H | OC₂H₅ | |
| 203 | 2 | N | C₂H₅ | OCH₃ | H | CF₃ | Fp.: 168°C |
| 204 | 2 | N | CH₃ | OC₂H₅ | H | CF₃ | Fp.: 137°C |
| 205 | 2 | N | CH₃ | N(CH₃)₂ | H | CF₃ | Fp.:93°C |
| 206 | 2 | N | SCH₃ | N(CH₃)₂ | H | CF₃ | Fp.:249°C |
| 207 | 2 | N | NHCH₃ | CN | H | CF₃ | Fp.:220°C |
| 208 | 2 | N | CH₃ | SCH₃ | H | CF₃ | Fp.:75°C |
| 209 | 2 | N | OCH₃ | 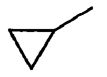 | H | CF₃ | Fp.: 189°C |
| 210 | 2 | N | SCH₃ | 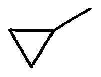 | H | CF₃ | Fp.:184°C |
| 211 | 2 | N | SCH₃ | C₃H₇-i | H | CF₃ | Fp.:86°C |
| 212 | 2 | N | SCH₃ | C₄H₉-t | H | CF₃ | Fp.:95°C |
| 213 | 2 | CH | Cl | Cl | H | CF₃ | Fp.:84°C |
| 214 | 2 | C-Cl | H | CH₃ | H | CF₃ | Fp.: 178°C |
| 215 | 2 | C-Br | CH₃ | SCH₃ | H | CF₃ | Fp.: 219°C |
| 216 | 2 | C-Br | CH₃ | OCH₃ | H | CF₃ | Fp.: 215°C |
| 217 | 2 | C-Cl | CH₃ | CH₃ | H | CF₃ | Fp.: 191°C |
| 218 | 2 | C-Cl | CH₃ | OCH₃ | H | CF₃ | Fp.: 229°C |
| 219 | 2 | CH | OC₂H₅ | OC₂H₅ | H | CF₃ | Fp.: 153°C |
| 220 | 2 | C-X | OCH₃ | R²+X: -(CH₂)₃ | H | CF₃ | Fp.:209°C |
| 221 | 2 | C-X | Cl | R²+X: -(CH₂)₃ | H | CF₃ | Fp.:212°C |
| 222 | 2 | C-CH₃ | CH₃ | OCH₃ | H | CF₃ | Fp.: 215°C |
| 223 | 2 | C-CH₃ | H | C₂H₅ | H | CF₃ | Fp.: 220°C |
| 224 | 2 | C-CH₃ | CH₃ | CH₃ | H | CF₃ | Fp.: 212°C |
| 225 | 3 | N | CH₃ | OCH₃ | H | CF₃ | Fp.: 208°C |
| | | | | | | | (Na-Salz) |
| 226 | 3 | N | OCH₃ | OCH₃ | CH₃ | CF₃ | Fp.: 174°C |

### Ausgangsstoffe der Formel (III):

### Beispiel (III-1)

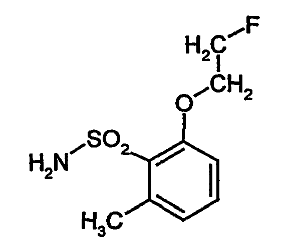

Eine Mischung aus 11,2 g (60 mMol) 2-Hydroxy-6-methyl-benzolsulfonamid, 10 g (78 mMol) 1-Brom-2-fluor-ethan, 16,6 g (120 mMol) Kaliumcarbonat und 350 ml Aceton wird 48 Stunden unter Rückfluß erhitzt und anschließend heiß filtriert. Das Filtrat wird unter vermindertem Druck eingeengt, der Rückstand in Methylenchlorid aufgenommen, mit Wasser gewaschen, mit Natriumsulfat getrocknet und filtriert Das Filtrat wird eingeengt, der Rückstand mit Diethylether digeriert und das kristallin angefallene Produkt durch Absaugen isoliert.

Man erhält 7,9 g (56 % der Theorie) 2-(2-Fluor-ethoxy)-6-methyl-benzolsulfonamid vom Schmelzpunkt 103°C.

Analog zu Beispiel (III-1) können beispielsweise auch die in der nachstehenden Tabelle 2 aufgeführten Verbindungen der allgemeinen Formel (III) hergestellt werden.

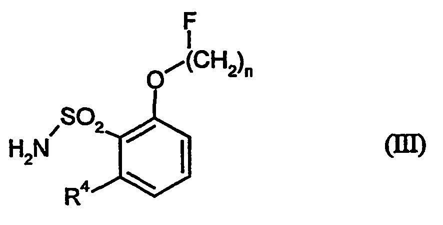

**Tabelle 2: Beispiele für die Verbindungen der Formel (III)**

| Bsp.-Nr. | n | R⁴ | Schmelzpunkt (°C) |
|---|---|---|---|
| III-2 | 2 | C₂H₅ | 108 |
| III-3 | 2 | CF₃ | |
| III-4 | 2 | C₃H₇-i | 114 |
| III-5 | 2 | OC₂H₅ | 140 |
| III-6 | 2 | C₃H₇-n | 117 |
| III-7 | 3 | C₃H₇-n | 108 |
| III-8 | 2 | Br | |
| III-9 | 3 | C₂H₅ | 92 |
| III-10 | 3 | CH₃ | 118 |
| III-11 | 3 | C₃H₇-i | |
| III-12 | 3 | CF₃ | |
| III-13 | 3 | Br | |
| III-14 | 3 | OCH₃ | |
| III-15 | 2 | OCH₃ | |
| III-16 | 2 | OC₃H₇-n | |
| III-17 | 2 | OC₃H₇-i | |
| III-18 | 3 | OC₂H₅ | |
| III-19 | 3 | OC₃H₇-n | |
| III-20 | 3 | OC₃H₇-i | |
| III-21 | 2 | CH₂CF₃ | |
| III-22 | 3 | CH₂CF₃ | |
| III-23 | 2 | Cl | |
| III-24 | 3 | Cl | |
| III-25 | 2 | Br | |
| III-26 | 3 | Br | |
| III27 | 2 | I | |
| III-28 | 3 | I | |

### Anwendungsbeisipiele:

### Beispiel A

| Pre-emergence-Test | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät Nach 24 Stunden wird der Boden so mit der Wirkstoffzubereitung besprüht, dass die jeweils gewünschte Wirkstoffmenge pro Flächeneinheit ausgebracht wird. Die Wirkstoffkonzentration in der Spritzbrühe wird so gewählt, dass in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, 2, 3, 4, 5, 6, 7, 8, 14, 15, 17, 20, 21, 24, 25 und 26 bei zum Teil guter Verträglichkeit gegenüber Kulturpflanzen, wie z.B. Weizen und Soja, sehr starke Wirkung gegen Unkräuter.

**Tabelle A1: Pre-emergence-Test Gewächshaus**

| Wirkstoff gemäß Herstellungsbeispiel-Nr. | Aufwandmenge (g a.i./ha) | Cyperus | Echinochloa | Abutilon | Galium | Solanum | Stellaria | Veronica |
|---|---|---|---|---|---|---|---|---|
| (3) | 8 | 100 | 100 | 100 | 100 | 100 | 100 | 100 |

**Tabelle A2: Pre-emergence-Test Gewächshaus**

| Wirkstoff gemäß Herstellungsbeispiel-Nr. | Aufwandmenge (g a.i./ha) | Weizen | Soja | Alopecurus | Sorghum | Chenopodium | Matricaria | Viola |
|---|---|---|---|---|---|---|---|---|
| (4) | 15 | 0 | 0 | 80 | 80 | 100 | 100 | 100 |

**Tabelle A3: Pre-emergence-Test Gewächshaus**

| Wirkstoff gemäß Herstellungsbeispiel-Nr. | Aufwandmenge (g a.i./ha) | Echinochloa | Amaranthus | Datura | Galium | Matricaria | Viola |
|---|---|---|---|---|---|---|---|
| (5) | 15 | 100 | 100 | 100 | 100 | 100 | 100 |

**Tabelle A4: Pre-emergence-Test Gewächshaus**

| Wirkstoff gemäß Herstellungsbeispiel-Nr. | Aufwandmenge (g a.i./ha) | Weizen | Echinochloa | Chenopodium | Galium | Stellaria | Viola |
|---|---|---|---|---|---|---|---|
| (7) | 30 | 0 | 90 | 90 | 90 | 100 | 100 |
| (8) | 30 | 0 | - | 90 | 90 | 100 | 100 |

**Tabelle A5: Pre-emergence-Test Gewächshaus**

| Wirkstoff gemäß Herstellungsbeispiel-Nr. | Aufwandmenge (g a.i./ha) | Weizen | Echinochloa | Chenopodium | Datura | Stellaria | Viola |
|---|---|---|---|---|---|---|---|
| (25) | 125 | 0 | 90 | 90 | 90 | 90 | 100 |

**Tabelle A6: Pre-emergence-Test Gewächshaus**

| Wirkstoff gemäß Herstellungsbeispiel-Nr. | Aufwandmenge (g a.i./ha) | Alopecurus | Echinochloa | Abutilon | Amaranthus | Galium | Sinapis |
|---|---|---|---|---|---|---|---|
| (14) | 60 | 100 | 100 | 95 | 95 | 90 | 95 |
| (15) | 60 | 95 | 95 | 95 | 100 | 95 | 95 |
| (17) | 60 | 95 | 95 | 95 | 95 | 95 | 90 |

**Tabelle A7: Pre-emergence-Test Gewächshaus**

| Wirkstoff gemäß Herstellungsbeispiel- | Aufwandmenge (g a.i./ha) | Alopecurus | Setaria | Abutilon | Amaranthus | Galium | Sinapis |
|---|---|---|---|---|---|---|---|
| (20) | 60 | 90 | 100 | 90 | 95 | 95 | 95 |
| (21) | 60 | 90 | 100 | 90 | 95 | 95 | 100 |
| (6) | 60 | 80 | 95 | 90 | 90 | 90 | 90 |

**Tabelle A8: Pre-emergence-Test Gewächshaus**

| Wirkstoff gemäß Herstellungsbeispiel-Nr. | Aufwandmenge (g a.i./ha) | Alopecurus | Avena fatua | Setaria | Amaranthus | Galium | Xanthium |
|---|---|---|---|---|---|---|---|
| (24) | 250 | 100 | 95 | 95 | 100 | 100 | 95 |
| (1) | 250 | 100 | 100 | 100 | 100 | 100 | 100 |
| (25) | 250 | 95 | 90 | 100 | 100 | 95 | 100 |
| (26) | 250 | 100 | 90 | 100 | 100 | 95 | 95 |
| (2) | 250 | 100 | 95 | 100 | 100 | 100 | 95 |

### Beispiel B

| Post-emergence-Test | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, dass die jeweils gewünschten Wirkstoffmengen pro Flächeneinheit ausgebracht werden. Die Konzentration der Spritzbrühe wird so gewählt, dass in 1000 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.
Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, 2, 3, 4, 5, 6, 7, 8, 12, 13, 14, 15, 17, 18, 20, 21, 22, 24, 25 und 26, bei zum Teil guter Verträglichkeit gegenüber Kulturpflanzen, wie z.B. Mais, Weizen und Zuckerrüben, sehr starke Wirkung gegen Unkräuter.

**Tabelle B1: Post-emergence-Test Gewächshaus**

| Wirkstoff gemäß Herstellungsbeispiel-Nr. | Aufwandmenge (g a.i./ha) | Alopecurus | Avena fatua | Setaria | Abutilon | Amaranthus | Galium | Sinapis |
|---|---|---|---|---|---|---|---|---|
| (24) | 250 | 100 | 90 | 95 | 100 | 100 | 90 | 100 |
| (1) | 250 | 95 | 95 | 100 | 100 | 100 | 100 | 100 |
| (25) | 250 | 90 | 90 | 100 | 100 | 100 | 100 | 100 |
| (26) | 250 | 95 | 90 | 90 | 100 | 100 | 90 | 95 |
| (2) | 250 | 100 | 100 | 100 | 100 | 100 | 100 | 100 |

**Tabelle B2: Post-emergence-Test Gewächshaus**

| 1 Wirkstoff gemäß Herstellungsbeispiel-Nr. | Aufwandmenge (g a.i./ha) | Weizen | Alopecuros | Abutilon | Amaranthus | Datura | Solanum |
|---|---|---|---|---|---|---|---|
| (25) | 125 | 10 | 90 | 95 | 100 | 100 | 100 |
| (7) | 30 | 0 | 90 | 100 | 100 | 100 | 100 |

**Tabelle B3: Post-emergence-Test Gewächshaus**

| Wirkstoff gemäß Herstellungsbeispiel-Nr. | Aufwandmenge (g a.i./ha) | Weizen | Lolium | Abutilon | Ipomoea | Matricaria | Solanum | Stellaria |
|---|---|---|---|---|---|---|---|---|
| (17) | 8 | 10 | 90 | 100 | 100 | 100 | 100 | 100 |

**Tabelle B4: Post-emergence-Test Gewächshaus**

| Wirkstoff gemäß Herstellungsbeispiel-Nr. | Aufwandmenge (g a.i./ha) | Weizen | Abutilon | Amaranthus | Matricaria | Xanthium |
|---|---|---|---|---|---|---|
| (18) | 15 | 15 | 90 | 95 | 95 | 90 |

**Tabelle B5: Post-emergence-Test Gewächshaus**

| Wirkstoff gemäß Herstellungsbeispiel-Nr. | Aufwandmenge (g a.i./ha) | Weizen | Zuckerrüben | Amaranthus | Ipomoea | Matricaria |
|---|---|---|---|---|---|---|
| (14) | 30 | 0 | 0 | 100 | 95 | 90 |

**Tabelle B6: Post-emergence-Test Gewächshaus**

| Wirkstoff gemäß Herstellungsbeispiel-Nr. | Aufwandmenge (g a.i./ha) | Weizen | Mais | Datura | Ipomoea | Matricaria | Solanum | Stellaria |
|---|---|---|---|---|---|---|---|---|
| (8) | 8 | 0 | 10 | 90 | 100 | 95 | 100 | 95 |

**Tabelle B7: Post-emergence-Test Gewächshaus**

| Wirkstoff gemäß Herstellungsbeispiel-Nr. | Aufwandmenge (g a.i./ha) | Alopecurus | Abutilon | Amaranthus | Ipomoea | Matricaria | Viola |
|---|---|---|---|---|---|---|---|
| (4) | 30 | 90 | 95 | 100 | 95 | 100 | 95 |

**Tabelle B8: Post-emergence-Test Gewächshaus**

| Wirkstoff gemäß Herstellungsbeispiel-Nr. | Aufwandmenge (g a.i./ha) | Alopecurus | Lolium | Abutilon | Amaranthus | Matricaria | Viola | Xanthium |
|---|---|---|---|---|---|---|---|---|
| (3) | 30 | 95 | 95 | 100 | 100 | 100 | 100 | 100 |

**Tabelle B9: Post-emergence-Test Gewächshaus**

| Wirkstoff gemäß Herstellungsbeispiel-Nr. | Aufwandmenge (g a.i./ha) | Alopecurus | Abutilon | Amaranthus | Ipomoea | Xanthium |
|---|---|---|---|---|---|---|
| (6) | 30 | 90 | 90 | 90 | 100 | 95 |

**Tabelle B10: Post-emergence-Test Gewächshaus**

| Wirkstoff gemäß Herstellungsbeispiel-Nr. | Aufwandmenge (g a.i./ha) | Alopecurus | Lolium | Abutilon | Amaranthus | Ipomoea | Matricaria | Stellaria |
|---|---|---|---|---|---|---|---|---|
| (5) | 30 | 95 | 95 | 100 | 100 | 100 | 100 | 100 |
| (13) | 30 | 90 | 90 | 100 | 100 | 90 | 100 | 100 |
| (15) | 30 | 90 | 90 | 95 | 100 | 100 | 100 | 100 |

**Tabelle B11: Post-emergence-Test Gewächshaus**

| Wirkstoff gemäß Herstellungsbeispiel-Nr. | Aufwandmenge (g a.i./ha) | Echinochloa | Abutilon | Amaranthus | Datura |
|---|---|---|---|---|---|
| (12) | 30 | 90 | 95 | 95 | 95 |

**Tabelle B12: Post-emergence-Test Gewächshaus**

| Wirkstoff gemäß Herstellungsbeispiel-Nr. | Aufwandmenge (g a.i./ha) | Echinchloa | Ipomoea | Matricaria | Polygonum | Xanthium |
|---|---|---|---|---|---|---|
| (20) | 30 | 90 | 100 | 95 | 95 | 100 |

**Tabelle B13: Post-emergence-Test Gewächshaus**

| Wirkstoff gemäß Herstellungsbeispiel- | Aufwandmenge (g a.i./ha) | Avena fatua | Lolium Nr. | Datura | Matricaria | Solanum |
|---|---|---|---|---|---|---|
| (21) | 30 | 100 | 100 | 100 | 100 | 100 |

**Tabelle B14: Post-emergence-Test Gewächshaus**

| Wirkstoff gemäß Herstellungsbeispiel-Nr. | Aufwandmenge (g a.i./ha) | Alopecurus | Setaria | Amaranthus | Galium | Ipomoea | Sinapis |
|---|---|---|---|---|---|---|---|
| (22) | 60 | 90 | 95 | 100 | 95 | 100 | 95 |

### Beispiel C

| Podosphaera-Test (Apfel) / protektiv | |
|---|---|
| Lösungsmittel : | 49 Gewichtsteile Aceton |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Apfelmehltauerregers ***Podosphaera leucotricha*** inokuliert. Die Pflanzen werden dann im Gewächshaus bei ca. 23°C und einer relativen Luftfeuchtigkeit von ca. 70 % aufgestellt

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen beispielsweise die Verbindungen gemäß Herstellungsbeispiel 1, 24, 25 und 26 bei einer Aufwandmenge von 100 g/ha einen Wirkungsgrad von 100 % gegenüber Apfelmehltau (Podosphaera leucotricha).

**TABELLE C**

| **Podosphaera-Test (ApfeI) / protektiv** | | |
|---|---|---|
| Wirkstoff gemäß Herstellungsbeispiel-Nr. | Aufwandmenge an Wirkstoff in g/ha | % Wirkungsgrad |
| 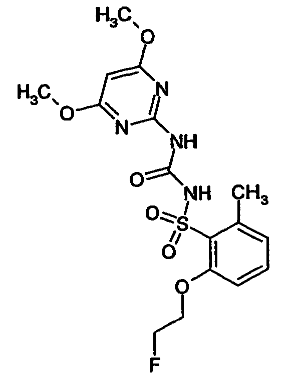 | | |
| (24) | 100 | 100 |
| 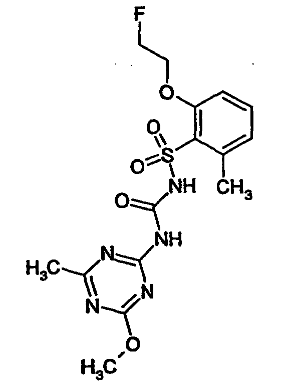 | | |
| (1) | 100 | 100 |
| 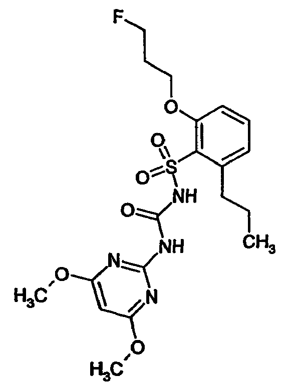 | | |
| (25) | 100 | 100 |
| 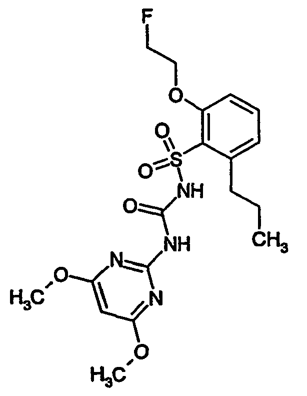 | | |
| (26) | 100 | 100 |

## Patentansprüche

1. Verbindungen der allgemeinen Formel (I) 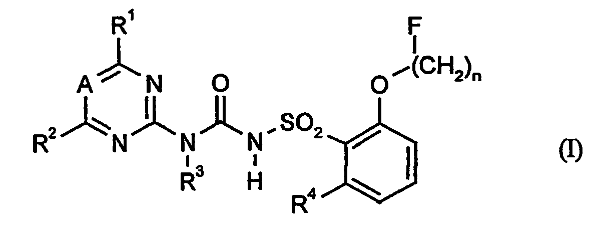 in welcher
n für die Zahlen 2, 3 oder 4 steht,
A für Stickstoff oder die Gruppierung C-X steht, wobei
X für Wasserstoff, Halogen oder jeweils gegebenenfalls durch Halogen substituiertes Alkyl oder Alkoxy mit jeweils 1 bis 4 Kohlenstoffatomen steht, oder zusammen mit R¹ oder R² für eine der Gruppierungen -CH₂CH₂O-, -CH₂CH₂S-, -CH₂CH₂CH₂- steht,
R¹ für Wasserstoff, für Halogen oder für jeweils gegebenenfalls durch Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen steht,
R² für Wasserstoff, für Cyano, für Halogen oder für jeweils gegebenenfalls durch Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, oder für Cycloalkyl mit 3 bis 6 Kohlenstoffatomen steht,
R³ für Wasserstoff oder für gegebenenfalls durch Cyano, Halogen, C₁-C₄-Alkoxy oder C₁-C₄-Alkoxy-carbonyl substituiertes Alkyl mit 1 bis 4 Kohlenstoffatomen steht, und
R⁴ für Halogen, für jeweils gegebenenfalls durch Cyano, Halogen oder C₁-C₄-Alkoxy substituiertes Alkyl oder Alkoxy mit jeweils 1 bis 4 Kohlenstoffatomen, für jeweils gegebenenfalls durch Cyano oder Halogen substituiertes Alkenyl, Alkinyl, Alkenyloxy oder Alkenyloxy mit jeweils 2 bis 4 Kohlenstoffatomen, oder für gegebenenfalls durch Cyano, Halogen oder C₁-C₄-Alkyl substituiertes Cycloalkyl mit 3 bis 6 Kohlenstoffatomen steht,
sowie Salze von Verbindungen der Formel (I).

2. Verbindungen gemäß Anspruch 1, **dadurch gekennzeichnet, dass**
n für die Zahlen 2, 3 oder 4 steht,
A für Stickstoff oder die Gruppierung C-X steht, wobei
X für Wasserstoff, Fluor, Chlor, Brom oder jeweils gegebenenfalls durch Fluor und/oder Chlor substituiertes Methyl, Ethyl, Methoxy oder Ethoxy steht, oder zusammen mit R¹ oder R² für eine der Gruppierungen -CH₂CH₂O-, -CH₂CH₂S-, -CH₂CH₂CH₂- steht,
R¹ für Wasserstoff Fluor, Chlor, Brom, oder für jeweils gegebenenfalls durch Fluor, Chlor, Methoxy, Ethoxy oder n- oder i-Propoxy substituiertes Methyl, Ethyl, n- oder i-Propyl, n-, i-, s- oder t-butyl Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino steht,
R² für Wasserstoff, oder für Cyano, Fluor, Chlor, Brom, oder für jeweils gegebenenfalls durch Fluor, Chlor, Methoxy, Ethoxy oder n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino, oder für Cyclopropyl, Cyclobutyl, Cyclopentyl oder Cyclohexyl steht,
R³ für Wasserstoff oder für jeweils gegebenenfalls durch Cyano, Fluor, Chlor, Methoxy, Ethoxy, n- oder i-Propoxy, Methoxycarbonyl, Ethoxycarbonyl, n- oder i-Propoxy-carbonyl substituiertes Methyl, Ethyl, n- oder i-Propyl, n-, i-, s- oder t-Butyl steht, und
R⁴ für Fluor, Chlor, Brom, oder für jeweils gegebenenfalls durch Fluor, Chlor, Brom, Methoxy, Ethoxy, n- oder i-Propoxy, n-, i-, s- oder t-Butoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, für jeweils gegebenenfalls durch Fluor, Chlor und/oder Brom substituiertes Ethenyl, Propenyl, Butenyl, Ethinyl, Propinyl, Butinyl, Propenyloxy, Butenyloxy, Propinyloxy oder Butinyloxy, oder für jeweils gegebenenfalls durch Fluor, Chlor oder Methyl substituiertes Cyclopropyl, Cyclobutyl oder Cyclopentyl steht.

3. Verbindungen gemäß Anspruch 1, **dadurch gekennzeichnet, dass**
n für die Zahlen 2, 3 oder 4 steht,
A für Stickstoff oder die Gruppierung C-X steht, wobei
X für Wasserstoff, Fluor, Chlor, Methyl oder Methoxy steht, oder zusammen mit R¹ oder R² für eine der Gruppierungen -CH₂CH₂O-, -CH₂CH₂S-, -CH₂CH₂CH₂- steht.
R¹ für Wasserstoff, Fluor, Chlor, Brom, oder für jeweils gegebenenfalls durch Fluor, Chlor, Methoxy, Ethoxy oder n- oder i-Propoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Wasserstoff Cyano, Fluor, Chlor, Brom, oder für jeweils gegebenenfalls durch Fluor, Chlor, Methoxy, Ethoxy oder n- oder i-Propoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino, oder für Cyclopropyl steht,
R³ für Wasserstoff oder für jeweils gegebenenfalls durch Cyano, Fluor, Chlor, Methoxy, Ethoxy, n- oder i-Propoxy, Methoxycarbonyl, Ethoxycarbonyl, n- oder i-Propoxy-carbonyl substituiertes Methyl, Ethyl, n- oder i-Propyl steht, und
R⁴ für Fluor, Chlor, Brom, oder für jeweils gegebenenfalls durch Fluor, Chlor, Methoxy, Ethoxy, n- oder i-Propoxy substituiertes Methyl, Ethyl, n- oder i-Propyl, Methoxy, Ethoxy, n- oder i-Propoxy, für je weils gegebenenfalls durch Fluor, Chlor und/oder Brom substituiertes Propenyl, Butenyl, Propinyl, Butinyl, Propenyloxy, Butenyloxy, Propinyloxy oder Butinyloxy, oder für gegebenenfalls durch Fluor, Chlor oder Methyl substituiertes Cyclopropyl steht

4. Verbindungen gemäß Anspruch 1, daurch **gekennzeichnet**, dass
n für die Zahl 2 oder 3 steht,
A für Stickstoff oder eine CH-Gruppierung steht,
R¹ für Cyano, Chlor, Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamino, Ethylamino, Dimethylamino oder Cyclopropyl steht,
R² für Chlor, Methyl, Trifluormethyl, Ethyl, Methoxy, Difluormethoxy, Trifluormethoxy, Ethoxy, Fluorethoxy, Difluorethoxy, Trifluorethoxy, Methylthio, Ethylthio, Methylamino, Ethylamino oder Dimethylamino steht,
R³ für Wasserstoff oder Methyl steht, und
R⁴ für Fluor, Chlor, Brom, Methyl, Trifluormethyl, Ethyl, n- oder i-Propyl, Methoxy, Ethoxy, n- oder i-Propoxy, oder für Cyclopropyl steht.

5. Verbindungen gemäß Anspruch 1, **dadurch gekennzeichnet, dass** n für 3 oder 4 steht.

6. Verfahren zum Herstellen von Verbindungen gemäß Anspruch 1, **dadurch gekennzeichnet, dass** substituierte Aminoazine der allgemeinen Formel (II) 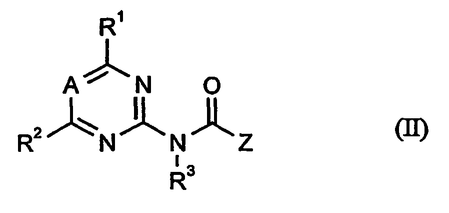 in welcher
A, R¹ und R² die in Anspruch 1 angegebene Bedeutung haben,
Z für Halogen, Alkoxy oder Aryloxy steht und
R³ die in Anspruch 1 angegebene Bedeutung hat oder für die Gruppierung -C(O)-Z steht,
mit Fluoralkoxybenzolsulfonamiden der allgemeinen Formel (III) 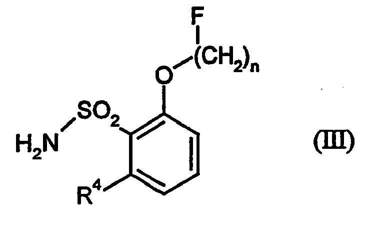 in welcher
n und R⁴ die in Anspruch 1 angegebene Bedeutung haben,
gegebenenfalls in Gegenwart eines oder mehrerer Reaktionshilfsmittel und gegebenenfalls in Gegenwart eines oder mehrerer Verdünnungsmittel umgesetzt werden,
und gegebenenfalls die so erhaltenen Verbindungen der allgemeinen Formel (I) nach üblichen Methoden in ihre Salze überführt werden.

7. Verfahren zum Bekämpfen von unerwünschtem Pflanzenwuchs oder Mikroorganismen, **dadurch gekennzeichnet, dass** man mindestens eine Verbindung gemäß einem der Ansprüche 1 bis 5 auf die unerwünschten Pflanzen, Makroorganismen und/oder ihren Lebensraum einwirken läßt.

8. Verwendung von mindestens einer Verbindung gemäß einem der Ansprüche 1 bis 5 zum Bekämpfen von unerwünschten Pflanzen oder Mikrorganismen.

9. Herbizides oder fungizides Mittel, **gekennzeichnet durch** einen Gehalt an einer Verbindung gemäß einem der Ansprüche 1 bis 5 und üblichen Streckmitteln und/oder oberflächenaktiven Mitteln.

10. Verbindungen der allgemeinen Formel (III) 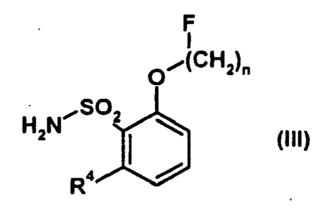 in welcher
n = 3 und R⁴ die in Anspruch 1 angegebene Bedeutung hat.

## Claims

1. A compound of the general formula (I) 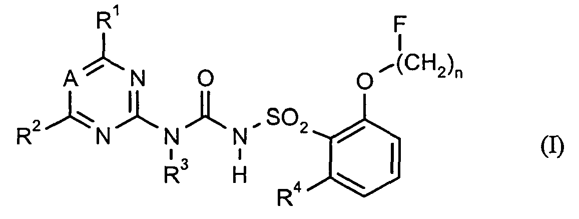 in which
n represents the numbers 2, 3 or 4,
A represents nitrogen or the grouping C-X, where
X represents hydrogen, halogen or in each case optionally halogen-substituted alkyl or alkoxy having in each case 1 to 4 carbon atoms, or together with R¹ or R² represents one of the groupings -CH₂CH₂O-, -CH₂CH₂S-, -CH₂CH₂CH₂-,
R¹ represents hydrogen, represents halogen or represents in each case optionally halogen- or C₁-C₄-alkoxy-substituted alkyl, alkoxy, alkylthio, alkylamino or dialkylamino having in each case 1 to 4 carbon atoms in the alkyl groups,
R² represents hydrogen, represents cyano, represents halogen or represents in each case optionally halogen- or C₁-C₄-alkoxy-substituted alkyl, alkoxy, alkylthio, alkylamino or dialkylamino having in each case 1 to 4 carbon atoms in the alkyl groups, or represents cycloalkyl having 3 to 6 carbon atoms,
R³ represents hydrogen or represents optionally cyano-, halogen-, C₁-C₄-alkoxy- or C₁-C₄-alkoxycarbonyl-substituted alkyl having 1 to 4 carbon atoms, and
R⁴ represents halogen, represents in each case optionally cyano-, halogen- or C₁-C₄-alkoxy-substituted alkyl or alkoxy having in each case 1 to 4 carbon atoms, represents in each case optionally cyano- or halogen-substituted alkenyl, alkynyl, alkenyloxy or alkynyloxy having in each case 2 to 4 carbon atoms, or represents optionally cyano-, halogen- or C₁-C₄-alkyl-substituted cycloalkyl having 3 to 6 carbon atoms,
or a salt of a compound of the formula (I).

2. A compound as claimed in claim 1, **characterized in that**
n represents the numbers 2, 3 or 4,
A represents nitrogen or the grouping C-X, where
X represents hydrogen, fluorine, chlorine, bromine or in each case optionally fluorine- and/or chlorine-substituted methyl, ethyl, methoxy or ethoxy, or together with R¹ or R² represents one of the groupings -CH_{z}CH₂O-, -CH₂CH₂S-, -CH₂CH₂CH₂-,
R¹ represents hydrogen, fluorine, chlorine, bromine, or represents in each case optionally fluorine-, chlorine-, methoxy-, ethoxy- or n- or i-propoxy-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, dimethylamino or diethylamino,
R² represents hydrogen, or represents cyano, fluorine, chlorine, bromine, or represents in each case optionally fluorine-, chlorine-, methoxy-, ethoxy- or n- or i-propoxy-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, dimethylamino or diethylamino, or represents cyclopropyl, cyclobutyl, cyclopentyl or cyclohexyl,
R³ represents hydrogen or represents in each case optionally cyano-, fluorine-, chlorine-, methoxy-, ethoxy-, n- or i-propoxy-, methoxycarbonyl-, ethoxycarbonyl-, n- or i-propoxycarbonyl-substituted methyl, ethyl, n- or i-propyl, n-, i-, s- or t-butyl, and
R⁴ represents fluorine, chlorine, bromine, or represents in each case optionally fluorine-, chlorine-, bromine-, methoxy-, ethoxy-, n- or i-propoxy-, n-, i-, s- or t-butoxy-substituted methyl, ethyl, n- or i-propyl, n-, i-, s- or t-butyl, methoxy, ethoxy, n- or i-propoxy, n-, i-, s- or t-butoxy, represents in each case optionally fluorine-, chlorine- and/or bromine-substituted ethenyl, propenyl, butenyl, ethynyl, propynyl, butynyl, propenyloxy, butenyloxy, propynyloxy or butynyloxy, or represents in each case optionally fluorine-, chlorine- or methyl-substituted cyclopropyl, cyclobutyl or cyclopentyl.

3. A compound as claimed in claim 1, **characterized in that**
n represents the numbers 2, 3 or 4,
A represents nitrogen or the grouping C-X, where
X represents hydrogen, fluorine, chlorine, methyl or methoxy, or together with R¹ or R² represents one of the groupings -CH₂CH₂O-, -CH₂CH₂S-, -CH₂CH₂CH₂-,
R¹ represents hydrogen, fluorine, chlorine, bromine, or represents in each case optionally fluorine-, chlorine-, methoxy-, ethoxy- or n- or i-propoxy-substituted methyl, ethyl, n- or i-propyl, methoxy, ethoxy, n-or i-propoxy, methylthio, ethylthio, n- or i-propylthio, methylamino, ethylamino, n- or i-propylamino, dimethylamino or diethylamino,
R² represents hydrogen, cyano, fluorine, chlorine, bromine, or represents in each case optionally fluorine-, chlorine-, methoxy-, ethoxy- or n- or i-propoxy-substituted methyl, ethyl, n- or i-propyl, methoxy, ethoxy, n- or i-propoxy, methylthio, ethylthio, n- or i-propylthio, methylamino, ethylamino, n- or i-propylamino, dimethylamino or diethylamino, or represents cyclopropyl,
R³ represents hydrogen or represents in each case optionally cyano-, fluorine-, chlorine-, methoxy-, ethoxy-, n- or i-propoxy-, methoxycarbonyl-, ethoxycarbonyl-, n- or i-propoxycarbonyl-substituted methyl, ethyl, n- or i-propyl, and
R⁴ represents fluorine, chlorine, bromine, or represents in each case optionally fluorine-, chlorine-, methoxy-, ethoxy-, n- or i-propoxy-substituted methyl, ethyl, n- or i-propyl, methoxy, ethoxy, n- or i-propoxy, represents in each case optionally fluorine-, chlorine- and/or bromine-substituted propenyl, butenyl, propynyl, butynyl, propenyloxy, butenyloxy, propynyloxy or butynyloxy, or represents optionally fluorine-, chlorine- or methyl-substituted cyclopropyl.

4. A compound as claimed in claim 1, **characterized in that**
n represents the number 2 or 3,
A represents nitrogen or a CH grouping,
R¹ represents cyano, chlorine, methyl, trifluoromethyl, ethyl, methoxy, difluoromethoxy, trifluoromethoxy, ethoxy, fluoroethoxy, difluoroethoxy, trifluoroethoxy, methylthio, ethylthio, methylamino, ethylamino, dimethylamino or cyclopropyl,
R² represents chlorine, methyl, trifluoromethyl, ethyl, methoxy, difluoromethoxy, trifluoromethoxy, ethoxy, fluoroethoxy, difluoroethoxy, trifluoroethoxy, methylthio, ethylthio, methylamino, ethylamino or dimethylamino,
R³ represents hydrogen or methyl, and
R⁴ represents fluorine, chlorine, bromine, methyl, trifluoromethyl, ethyl, n- or i-propyl, methoxy, ethoxy, n- or i-propoxy, or represents cyclopropyl.

5. A compound as claimed in claim 1, **characterized in that** n represents 3 or 4.

6. A process for preparing compounds as claimed in claim 1, **characterized in that** substituted aminoazines of the general formula (II) 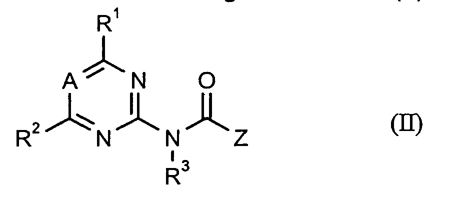 in which
A, R¹ and R² are as defined in claim 1,
Z represents halogen, alkoxy or aryloxy and
R³ is as defined in claim 1 or represents the grouping-C(O)-Z,
are reacted with fluoroalkoxybenzenesulfonamides of the general formula (III) 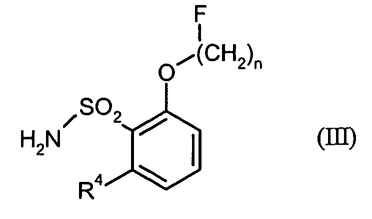 in which
n and R⁴ are as defined in claim 1,
if appropriate in the presence of one or more reaction auxiliaries and if appropriate in the presence of one or more diluents,
and the resulting compounds of the general formula (I) are, if appropriate, converted into their salts using customary methods.

7. A method for controlling unwanted vegetation or microorganisms, **characterized in that** at least one compound as claimed in any of claims 1 to 5 is allowed to act on the unwanted plants, microorganisms and/or their habitat.

8. The use of at least one compound as claimed in any of claims 1 to 5 for controlling unwanted plants or microorganisms.

9. A herbicidal or fungicidal composition, **characterized in that** it comprises a compound as claimed in any of claims 1 to 5 and customary extenders and/or surfactants.

10. A compound of the general formula (III) 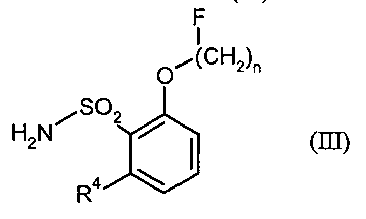 in which
n is 3 and R⁴ is as defined in claim 1.

## Revendications

1. Composés de formule générale (I) 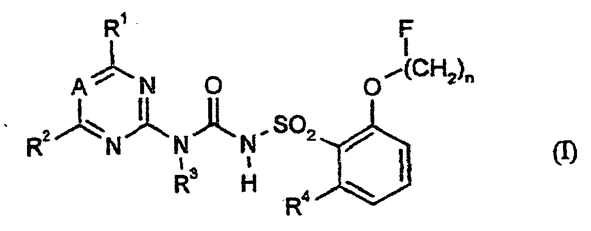 dans laquelle
n est un nombre 2, 3 ou 4,
A représente un atome d'azote ou le groupement C-X, où
X représente un atome d'hydrogène, un atome d'halogène ou un groupe alkyle ou alkoxy ayant chacun de 1 à 4 atomes de carbone, éventuellement substitué par un substituant halogène, ou conjointement avec R¹ ou R² représente un des groupements -CH₂CH₂O-, -CH₂CH₂S-, -CH₂CH₂CH₂-,
R¹ représente un atome d'hydrogène, un atome d'halogène ou un groupe alkyle, alkoxy, alkylthio, alkylamino ou dialkylamino ayant chacun de 1 à 4 atomes de carbone dans les groupes alkyle, éventuellement substitué chacun par un substituant halogène ou alkoxy en C₁-C₄,
R² représente un atome d'hydrogène, un groupe cyano, un atome d'halogène ou un groupe alkyle, alkoxy, alkylthio, alkylamino ou dialkylamino ayant chacun de 1 à 4 atomes de carbone dans les groupes alkyle, éventuellement substitué chacun par un substituant halogène ou alkoxy en C₁-C₄, ou un groupe cycloalkyle ayant 3 à 6 atomes de carbone,
R³ représente un atome d'hydrogène ou un groupe alkyle ayant de 1 à 4 atomes de carbone, éventuellement substitué par un substituant cyano, halogène, alkoxy en C₁-C₄ ou (alkoxy en C₁-C₄) carbonyle, et
R⁴ représente un atome d'halogène, un groupe alkyle ou alkoxy ayant chacun de 1 à 4 atomes de carbone, éventuellement substitué chacun par un substituant cyano, halogène ou alkoxy en C₁-C₄, un groupe alcényle, alcynyle, alcényloxy ou alcynyloxy ayant chacun de 2 à 4 atomes de carbone, éventuellement substitué chacun par un substituant cyano ou halogène, ou un groupe cycloalkyle ayant de 3 à 6 atomes de carbone, éventuellement substitué par un substituant cyano, halogène ou alkyle en C₁-C₄,
ainsi que des sels des composés de formule (I).

2. Composés selon la revendication 1, **caractérisés en ce que**
n est un nombre 2, 3 ou 4,
A représente un atome d'azote ou le groupement C-X, où
x représente un atome d'hydrogène, de fluor, de chlore, de brome ou un groupe méthyle, éthyle, méthoxy ou éthoxy, éventuellement substitué chacun par un substituant fluor et/ou chlore, ou conjointement avec R¹ ou R² représente un des groupements -CH₂CH₂O-, -CH₂CH₂S-, -CH₂CH₂CH₂-,
R¹ représente un atome d'hydrogène, de fluor, de chlore, de brome ou un groupe méthyle, éthyle, n- ou i-propyle, n-, i-, s- ou t-butyle, méthoxy, éthoxy ou n- ou i-propoxy, n-, i-, s- ou t-butoxy, méthylthio, éthylthio, n- ou i-propylthio, n-, i-, s- ou t-butylthio, méthylamino, éthylamino, n- ou i-propylamino, n-, i-, s- ou t-butylamino, diméthylamino ou diéthylamino, éventuellement substitué chacun par un substituant fluor, chlore, méthoxy, éthoxy ou n- ou i-propoxy,
R² représente un atome d'hydrogène ou un groupe cyano, un atome de fluor, de chlore, de brome, ou un groupe méthyle, éthyle, n- ou i-propyle, n-, i-, s- ou t-butyle, méthoxy, éthoxy, n- ou i-propoxy, n-, i-, s- ou t-butoxy, méthylthio, éthylthio, n- ou i-propylthio, n-, i-, s- ou t-butylthio, méthylamino, éthylamino, n- ou i-propylamino, n-, i-, s- ou t-butylamino, diméthylamino ou diéthylamino, éventuellement substitué chacun par un substituant fluor, chlore, méthoxy, éthoxy ou n- ou i-propoxy, ou un groupe cyclopropyle, cyclobutyle, cyclopentyle ou cyclohexyle,
R³ représente un atome d'hydrogène ou un groupe méthyle, éthyle, n- ou i-propyle, n-, i-, s- ou t-butyle, éventuellement substitué chacun par un substituant cyano, fluor, chlore, méthoxy, éthoxy, n- ou i-propoxy, méthoxycarbonyle, éthoxycarbonyle, n- ou i-propoxycarbonyle, et
R⁴ représente un atome de fluor, de chlore, de brome, ou un groupe méthyle, éthyle, n- ou i-propyle, n-, i-, s- ou t-butyle, méthoxy, éthoxy, n- ou i-propoxy, n-, i-, s- ou t-butoxy, éventuellement substitué chacun par un substituant fluor, chlore, brome, méthoxy, éthoxy, n- ou i-propoxy, n-, i-, s- ou t-butoxy, un groupe éthényle, propényle, butényle, éthynyle, propynyle, butynyle, propényloxy, butényloxy, propynyloxy ou butynyloxy, éventuellement substitué chacun par un substituant fluor, chlore et/ou brome, ou un groupe cyclopropyle, cyclobutyle ou cyclopentyle, éventuellement substitué chacun par un substituant fluor, chlore ou méthyle.

3. Composés selon la revendication 1, **caractérisés en ce que**
n est un nombre 2, 3 ou 4,
A représente un atome d'azote ou le groupement C-X, où
x représente un atome d'hydrogène, de fluor, de chlore, un groupe méthyle ou méthoxy, ou conjointement avec R¹ ou R² représente un des groupements -CH₂CH₂O-, -CH₂CH₂S-, -CH₂CH₂CH₂-,
R¹ représente un atome d'hydrogène, de fluor, de chlore, de brome, ou un groupe méthyle, éthyle, n- ou i-propyle, méthoxy, éthoxy, n- ou i-propoxy, méthylthio, éthylthio, n- ou i-propylthio, méthylamino, éthylamino, n- ou i-propylamino, diméthylamino ou diéthylamino, éventuellement substitué chacun par un substituant fluor, chlore, méthoxy, éthoxy ou n- ou i-propoxy,
R² représente un atome d'hydrogène, un groupe cyano, un atome de fluor, de chlore, de brome, ou un groupe méthyle, éthyle, n- ou i-propyle, méthoxy, éthoxy, n- ou i-propoxy, méthylthio, éthylthio, n- ou i-propylthio, méthylamino, éthylamino, n- ou i-propylamino, diméthylamino ou diéthylamino, éventuellement substitué chacun par un substituant fluor, chlore, méthoxy, éthoxy ou n- ou i-propoxy, ou un groupe cyclopropyle,
R³ représente un atome d'hydrogène ou un groupe méthyle, éthyle, n- ou i-propyle, éventuellement substitué chacun par un substituant cyano, fluor, chlore, méthoxy, éthoxy, n- ou i-propoxy, méthoxycarbonyle, éthoxycarbonyle, n- ou i-propoxycarbonyle,
R⁴ représente un atome de fluor, de chlore, de brome, ou un groupe méthyle, éthyle, n- ou i-propyle, méthoxy, éthoxy, n- ou i-propoxy, éventuellement substitué chacun par un substituant fluor, chlore, méthoxy, éthoxy, n- ou i-propoxy, un groupe propényle, butényle, propynyle, butynyle, propényloxy, butényloxy, propynyloxy ou butynyloxy, éventuellement substitués chacun par un substituant fluor, chlore et/ou brome, ou un groupe cyclopropyle éventuellement substitué par un substituant fluor, chlore ou méthyle.

4. Composés selon la revendication 1, **caractérisés en ce que**
n est un nombre 2 ou 3,
A représente un atome d'azote ou un groupement CH,
R¹ représente un groupe cyano, chlore, méthyle, trifluorométhyle, éthyle, méthoxy, difluorométhoxy, trifluorométhoxy, éthoxy, fluoroéthoxy, difluoroéthoxy, trifluoroéthoxy, méthylthio, éthylthio, méthylamino, éthylamino, diméthylamino ou cyclopropyle,
R² représente un atome de chlore, un groupe méthyle, trifluorométhyle, éthyle, méthoxy, difluorométhoxy, trifluorométhoxy, éthoxy, fluoroéthoxy, difluoroéthoxy, trifluoroéthoxy, méthylthio, éthylthio, méthylamino, éthylamino ou diméthylamino,
R³ représente un atome d'hydrogène ou un groupe méthyle, et
R⁴ représente un atome de fluor, de chlore, de brome, un groupe méthyle, trifluorométhyle, éthyle, n- ou i-propyle, méthoxy, éthoxy, n- ou i-propoxy ou cyclopropyle.

5. Composés selon la revendication 1, **caractérisés en ce que** n vaut 3 ou 4.

6. Procédé pour la préparation de composés selon la revendication 1, **caractérisé en ce qu'**on fait réagir des aminoazines de formule générale (I) 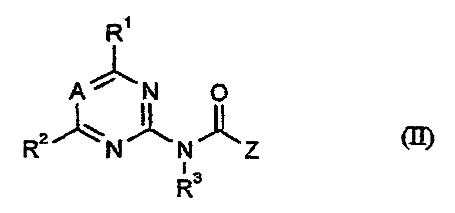 dans laquelle
A, R¹ et R² ont les significations données dans la revendication 1,
Z représente un atome d'halogène, un groupe allyloxy ou aryloxy et
R³ possède la signification donnée dans la revendication 1 ou représente le groupement -C(O)-Z,
avec des fluoroalkoxybenzènesulfonamides de formule (III) 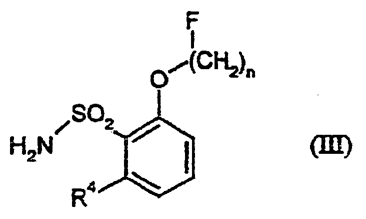 dans laquelle
n et R⁴ ont les significations données dans la revendication 1,
éventuellement en présence d'un ou plusieurs adjuvants et éventuellement en présence d'un ou plusieurs diluants,
et les composés ainsi obtenus de formule générale (I) sont éventuellement convertis en leurs sels à l'aide de procédés usuels.

7. Procédé pour la lutte contre une croissance végétale ou des microorganismes indésirables, **caractérisé en ce qu'**on laisse agir au moins un composé selon l'une des revendications 1 à 5 sur les plantes, les microorganismes indésirables et/ou leur environnement.

8. Utilisation d'au moins un composé selon l'une des revendications 1 à 5, pour la lutte contre les plantes ou microorganismes indésirables.

9. Agent herbicide ou fongicide, **caractérisé par** une teneur en un composé selon l'une des revendications 1 à 5, et en diluants et/ou agents tensio-actifs usuels.

10. Composés de formule générale (III) 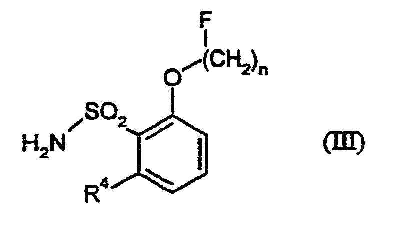 dans laquelle
n = 3 et R⁴ possède la signification donnée dans la revendication 1.
